# EUROPEAN PATENT SPECIFICATION

(11) **EP 1 775 588 B1**
(45) Date of publication and mention of the grant of the patent: **25.06.2008**
(21) Application number: 06122240.2
(22) Date of filing: 13.10.2006
(51) Int. Cl.: G01N 33/50, C12Q 1/25

(54) **Method for detecting the effect of a toxic substance using sub-mitochondrial particle**
Verfahren zum Nachweis der Wirkung einer toxischen Substanz unter Verwendung von submitochondrialen Partikeln
Procédé de détection de l'effet d'une substance toxique à l'aide d'une particule sous-mitochondriale

(30) Priority: 14.10.2005 KR 20050097098
(43) Date of publication of application: 18.04.2007
(73) Proprietor: Samsung Electronics Co., Ltd., Yeongtong-gu Suwon-si, Gyeonggi-do (KR)
(72) Inventor: Choi, Soo Hyung 215-502 Shinyeongtong Hyundai Apt, Gyeonggi-do, 445-983 (KR); Han, Jung Im, Gwanak-gu Seoul, 151-847 (KR)
(74) Representative: Grünecker, Kinkeldey, Stockmair & Schwanhäusser Anwaltssozietät

(56) References cited:
- EP-A- 0 228 290
- KNOBELOCH L. M. ET AL: "Use of submitochondrial particles for prediction of chemical toxicity in man" BULL. ENVIRON. CONTAM. TOXICOL., vol. 44, 1990, pages 661-668, XP009075526 New York
- ARGESE, E. ET AL: "Submitochondrial particles as in vitro biosensors of heavy metal toxicity" JOURNAL OF AQUATIC ECOSYSTEM HEALTH, vol. 5, 1996, pages 125-134, XP009075519 Netherlands
- DOHERTY, F. G. ET AL: "Repeatability of the submitochondrial particlde assay" ECOTOXICOLGY AND ENVIRONMENTAL SAFETY, vol. 53, 2002, pages 122-128, XP009075512 USA

## Description

### BACKGROUND OF THE INVENTION

### 1. Field of The Invention

The present invention generally relates to a method for detecting the effect of a toxic substance, and more particularly, to a method for detecting the effect of a toxic substance by monitoring a change in pH caused by the presence of sub-mitochondrial particles (SMP).

### 2. Description of the Related Art

Both, the human body as well as the environment have been threatened with a new form of damage (e.g., the danger of endocrine disruptor) due to the increase in the amount and type of toxic substances as a result of industrialization, ecosystem destruction, severity of pesticide residue in foods, heavy metal pollution in soil, and so on.

It has been therefore desirable to monitor the concentration of such chemicals in the environment, such that their deleterious influences upon the human body and the environment are minimized.

More than eighty thousand chemicals have been commercially all over the world, and several thousands of new chemicals are annually developed and used as raw materials for foods, medicines, cosmetics, medical instruments and the like. Therefore, in order to know how harmful such chemicals are to human body and environment, it is very important to exactly examine the influence of toxicity and the extent of the exposure thereof

An environmental risk assessment has been suggested as a part of the methods for securing the safety of these chemicals. For this assessment, legally required test items relating to environmental toxicity have been established. These test items examine and analyze the risk of chemicals directly and indirectly on the human body and the environment. Through such an environmental risk assessment, all information pertaining to certain chemical compounds such as the amount of exposure, degradation rate, concentration rate, toxicity and on the like, are analyzed and collected, which makes it possible to reasonably evaluate that compound and deduce an appropriate management method in the event of a problem.

To diagnose such an environmental risk at an early stage and cope with any relevant problem rapidly, there is need for a widespread environmental monitoring method. For example, toxicity may be measured by several methods that employ recombinant microorganisms, luminous bacteria, yeasts or enzymes. These methods mainly measure the environmental risk by using an optical technique. The major drawback of this optical technique is that complex and expensive analytical instruments are required.

U.S. Patent No. 5,278,048 discloses a method of measuring a pH change of 0.1 to 0.5 units within 1 to 4 minutes by attaching prokaryotic or eukaryotic cells to the surface of a silicon sensor installed in a micro chamber and bringing a cell affecting agent into contact with the silicon sensor.

However, since this patent employs the cell itself, the pH range capable of being changed by the presence of a toxic substance is confined to the maximum pH range represented by the cell, which causes a problem that the pH range to be measured is too narrow.

### SUMMARY OF THE INVENTION

The present inventors have therefore endeavored to overcome these problems and found that using the characteristics of sub-mitochondrial particle (SMP) in which the inner mitochondrial membrane is inverted to expose the innermost surface makes it possible to control an electron transport system of SMP through an external electron donor, which effectively measures a pH change caused by the presence of a toxic substance.

Therefore, the present invention provides a method for evaluating the effect of a toxic substance by measuring a pH change caused by SMP. Specifically, the method for detecting the effect of a toxic substance employing SMP is by measuring an external pH change caused due to contact between the SMP and the toxic substance. The contact between the SMP and the toxic substance inhibits reactions from receiving electrons from an external electron donor. This lack of electrons is communicated to an electron transport system, which in turn suppresses the introduction of protons from outside the SMP. In this manner, the the effect of a toxic substance can be measured.

The present invention also provides a method for rapidly and accurately detecting the effect of a toxic substance.

In addition, the present invention provides a method for detecting the effect of a toxic substance, which has a wide applicability to the detection of various kinds of toxic substances.

The other advantages of the invention will be understood by the following description and will also be appreciated by the embodiments of the invention described in detail below. Further, the advantages of the invention will readily be seen from the claims appended below.

### BRIEF DESCRIPTION OF DRAWINGS

FIG 1 is a diagram schematically illustrating the procedures of an electron transport system along with proton pumping by SMP; FIG 2 is a diagram schematically illustrating that when an electron transport system of SMP is inhibited by a toxic substance, an electron transport from an external electron donor is suppressed simultaneously with the obstruction of proton pumping; FIG 3(a) to (c) are graphs comparing the results of a pH change caused by SMP measured after treating the SMP with 10 weight percent (wt%) and 20 wt% ethanol as a toxic substance, respectively, with that of a control;
FIG 4(a) to (c) are graphs comparing the results of a pH change caused by SMP which are measured after treating with 20 wt% sorbitol as a non-toxic substance with that of a control;
FIG 5 is a graph representing the difference in pH change caused by each treatment of a control, 10% ethanol, 20% ethanol and 20% sorbitol as a pH change rate per hour by combining the graphs of FIG 3(a) to (c) illustrating the results of Example 1 and the graphs of FIG 4(a) to (c) illustrating the results of Example 2 together;
FIG 6 is a bar graph showing that the pH change caused by SMP can be regulated by controlling the amount of an electron donor supplied from the outside, wherein the pH change caused outside of the SMP varies as the concentration of NADH added; and
FIG 7 is a diagram hypothetically illustrating that the pH change caused by SMP due to the presence of a toxic substance can be indicated by a color change using a pH indicative dye.

### DETAILED DESCRIPTION OF THE INVENTION

The invention now will be described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

The terminology used herein is for the purpose of describing particular embodiments only and is not intended to be limiting of the invention. The terms "a" and "an" do not denote a limitation of quantity, but rather denote the presence of at least one of the referenced item. The term "or" means "and/or". The terms "comprising", "having", "including", and "containing" are to be construed as openended terms (i.e., meaning "including, but not limited to").

To accomplish the above invention, there is provided a method for detecting the effect of a toxic substance, which comprises the step of measuring a pH change caused by contacting an external electron donor having an electron transport system with a sub-mitochondrial particle (SMP) when the SMP contacts the sample.

The term "sub-mitochondrial particle (SMP)" used herein refers to small vesicles formed by sonicating mitochondria, in which the inner mitochondrial membrane is inverted to expose the innermost surface. Since the inner and outer membranes of mitochondria are reversed, adenosine triphosphates (ATPs) synthesized in an electron transport system of the SMP are excreted outside the SMP, and protons, namely, hydrogen ions (hereinafter, unified as "protons") that can be used for the synthesis of ATP are introduced into the inside of the SMP. The SMP, thus, receives electrons from an external electron donor and transports these electrons through an electron transport system. As a result of the protons are pumped from the outside of SMP into the inside and ATP is synthesized at the ATPase of the SMP. ATPases are a class of enzymes that catalyze the decomposition of adenosine triphosphate (ATP) into adenosine diphosphate (ADP) and a free phosphate ion. This dephosphorylation reaction releases energy, which the enzyme (in most cases) harnesses to drive other chemical reactions that would not otherwise occur.

SMPs used in the present invention can be available from the commercialized SMPs or be formed by the direct sonicating of mitochondria.

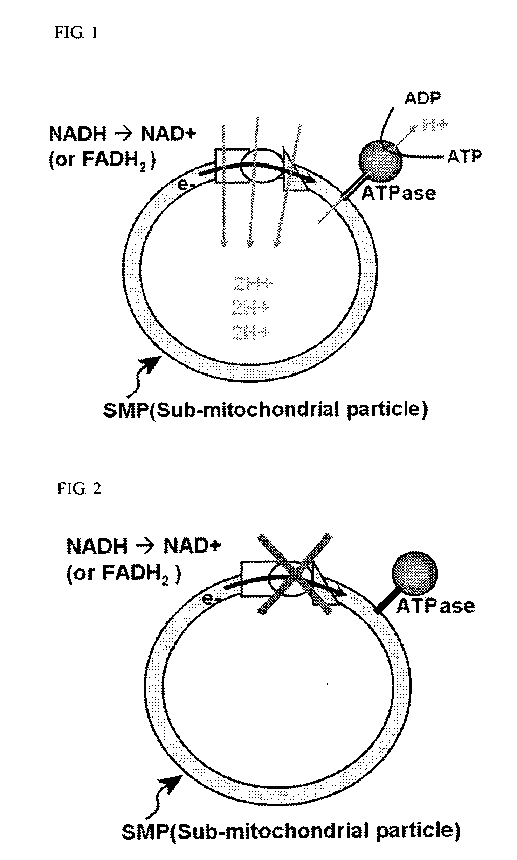

As described in FIG 1, the present invention employs the following phenomenon: when ATP is synthesized by the electron transport system of the SMP, protons are introduced into the inside of SMP from the outside. The electron transport system of the SMP synthesizes ATP using the introduced protons. The ATP is excreted outside the SMP. This results in a decrease in the number of protons existing outside the SMP which gradually increases the pH of the material outside the SMP. However, when SMP comes into contact with a toxic substance, as shown in FIG 2, the electron transport system of SMP is inhibited and thus the amount of protons introduced into the inside of SMP from the outside thereof is decreased. As a result, this causes almost no change in the number of protons existing outside of the SMP, thereby causing no pH increase outside the SMP.

The method of the present invention therefore involves brings the SMP into contact with a sample containing a target compound to be measured. Such contact may be conducted by preparing the SMP to be in a stock solution and mixing the sample with a suitable amount of the SMP stock solution.

Here, the sample used in the present invention is a fluid, for example, a solution prepared by dissolving a target compound to be measured in a suitable solution. Alternatively, target compound to be measured itself may be a fluid.

After mixing SMP with the sample, an external electron donor is finally added thereto, and thus, the pH change caused by the SMP is monitored, which makes it possible to measure the extent of pH change inhibited by the presence of the toxic substance.

Here, the external electron donor, which can induce a proton pumping by supplying electrons to the electron transport system of SMP, is preferably one or more selected from the group consisting of NADH, and FADH₂.

NADH is the reduced form of NAD+ (nicotinamide adenine dinucleotide). It forms NADP (nicotinamide adenine dinucleotide phosphate) with the addition of a phosphate group to the 2' position of an adenosyl nucleotide through an ester linkage.

Flavin is a tricyclic heteronuclear organic ring based on pteridine whose biochemical source is the vitamin riboflavin. The flavin moiety is often attached with an adenosine diphosphate to form flavin adenine dinucleotide (FAD), and in other circumstances, is found as flavin mononucleotide (or FMN), a phosphorylated form of riboflavin. The flavin group is capable of undergoing oxidation-reduction reactions, and can accept either one electron in a two step process or can accept two electrons at once. In the form of FADH₂, it is one of the cofactors that can transfer electrons to the electron transport system of the SMP.

As a result, the pH change depends on the concentration of the external electron donor. Namely, the higher the concentration of the external electron donor is, the larger the amount of protons introduced into the inside from the outside through the electron transport system of the SMP. This increases the reduction in the amount of protons and in thus enlarges the range of the pH increase. Accordingly, if the concentration of the external electron donor is controlled, it is possible to artificially regulate the amount of protons supplied to the electron transport system of SMP, which thus regulates the range of pH change in the outside of SMP.

The range of pH change occurring outside the SMP may differ depending on whether or not a toxic substance is included in the sample in contact with the SMP and the extent of the toxicity thereof Thus, in measuring the the pH change, it is possible to sensitively measure whether or not a toxic substance is included in the sample contacted with SMP (or whether or not the sample itself acts as a toxic substance) and the extent of toxicity thereof

According to the method of present invention, the pH change occuring outside the SMP can be detected by measuring the amount of protons as an electrical signal. Here, the electrical signal can be measured by using an electrode or a transistor. The measure signals can be one or more signals selected from the group consisting of of electrical current, electric potential, impedance and capacitance. For such electrical measurements, an amperometric biosensor, a potentiometric biosensor, or an impediometric biosensor known in the art may be employed.

The pH change can also be measured using a color indicator, wherein the change of color indicates the pH change.

The present invention will now be described in detail with reference to the following examples, which are not intended to limit the scope of the present invention.

### Example 1

First of all, a large quantity of SMPs were prepared by sonicating mitochondria.

Thereafter, the test tubes 1 to 3 were prepared, wherein 10 ml of a 2 mM KC1 solution (control solution) was added to the test tube 1, 10 ml of 10% ethanol dissolved in the control solution was added to the test tube 2, and 10 ml of 20% ethanol dissolved in the same was added to the test tube 3. SMPs prepared above were added to each test tube at a final concentration of 1.4 mg/ml.

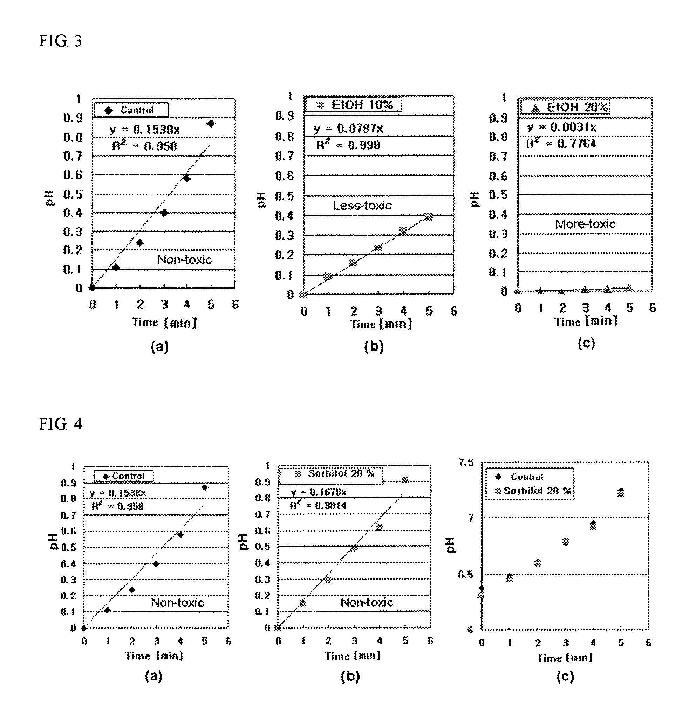

Next, 0.1 mM of NADH that acts as an external electron donor was added to each test tube at the same time, and the pH change in each test tube was measured for 5 minutes by using a pH meter. The results are shown in FIGs. 3(a) to 3(c).

FIG 3(a) is a graph illustrating the measured result of a pH change of the test tube 1 which contains only SMP and NADH in the control solution. It was found that electrons supplied by NADH are introduced into the inside of SMP and used in an electron transport system of SMP, which decreases the number of protons existing in the buffer and thus increases pH by about 0.9 for 5 minutes.

FIG 3(b) is a graph illustrating the measured result of a pH change of the test tube 2 that contains SMP and NADH in the solution dissolved with 10% ethanol. FIG 3(b) shows that since the electron transport system of SMP is inhibited by ethanol (toxic substance), the amount of electrons, which are supplied by NADH and then introduced into the inside of SMP, is decreased, and thus, the number of protons existing in the solution is not so decreased, thereby increasing pH by only about 0.4 during the 5 minutes of measurement.

FIG 3(c) is a graph illustrating the measured result of a pH change of the test tube 3 that contains SMP and NADH in the solution dissolved with 20% ethanol. It was found that the higher the concentration of ethanol is, the more severe the inhibition on the electron transport system of SMP is. There is therefore almost no pH change for 5 minutes.

### Example 2

In order to examine whether the method of the present invention is sensitive to only a toxic substance, the experiment was carried out according to the same method as described in Example 1 except that the sample prepared by dissolving 20% sorbitol in a 2 mM KCl solution was used.

The results are shown in FIGs. 4(a) to (c). Referring to the FIG's. 4(a) to (c), it was found that in case of treating the sample with a non-toxic compound, the pH change is almost identical to that of a control. These results suggest that the method for detecting the effect of a toxic substance using SMP according the present invention can sensitively respond to only a toxic substance and effectively detect the pH change caused by the presence thereof

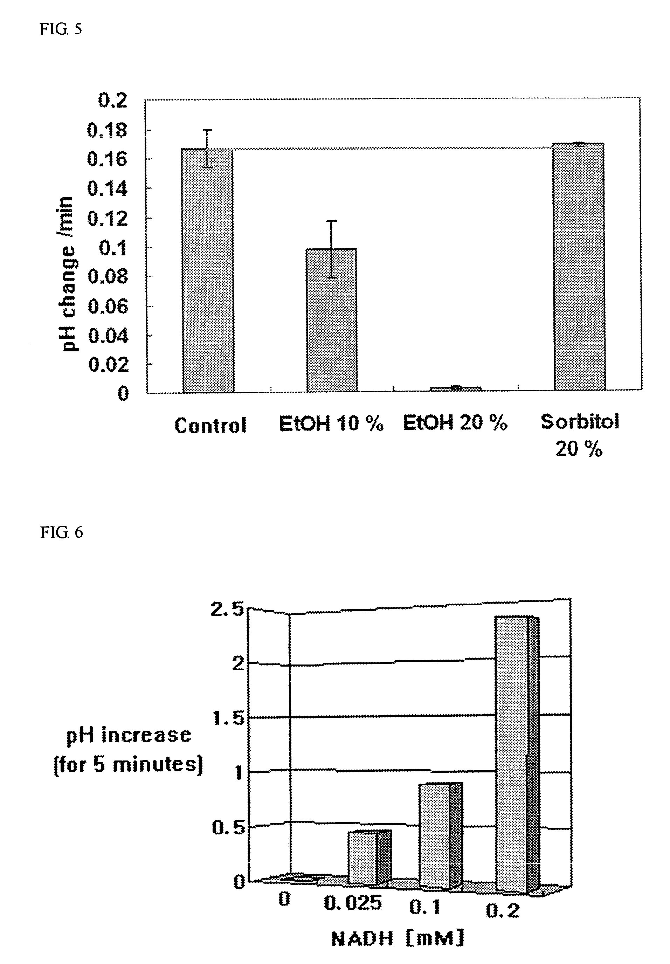

Referring to FIG 5 which exemplifies a bar graph showing the difference in a pH change as a pH change rate per hour by combining the results of Examples 1 and 2, the pH change rate in the treatment with a non-toxic substance was about 0.17 per minute which was identical to the control (that is, no toxicity was detected). However, in case of treating with a relatively weak toxic substance such as 10% ethanol, the pH change rate was lower than 0.1 per minute, and in case of treating with a relatively strong toxic substance, which was significantly lower than 0.02 per minute, implying that there was almost no pH change (toxicity detection).

Accordingly, it was confirmed that, in a system that employs the method of the present invention, it is possible to detect whether or not a sample has toxicity and even the intensity of its toxicity based on the range of pH change measured under the condition that SMP and the sample are contacted with each other.

### Example 3

In order to estimate the possibility of regulating the pH change caused outside the SMP by artificially controlling the amount of electrons supplied into an electron transport system of SMP, the experiment was performed in a manner similar to that described in Example 1 except that the concentration of NADH added into the control of Example 1. The NADH added into the control of Example 1 and was adjusted to 0 mM, 0.025 mM, 0.1 mM and 0.2 mM, respectively.

The results are shown in FIG 6. Referring to FIG. 6, it was found that depending on the concentration of an external electron donor, i.e., NADH, the pH change range increased for 5 minutes. The increase in the pH was significantly larger than the proportional increase to the concentration of NADH. These results suggest that it is possible to regulate the pH change occurring outside the SMP by controlling the concentration of the external electron donor.

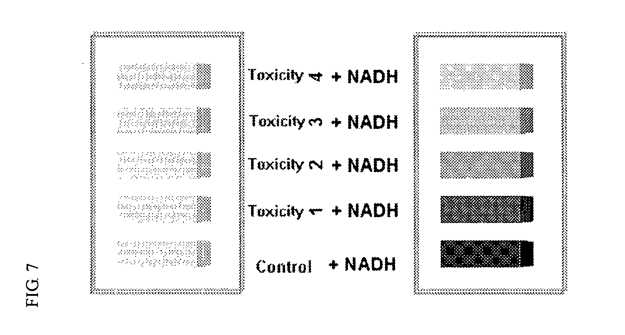

Meanwhile, FIG 7 is a diagram hypothetically illustrating that the pH change caused by the SMP depending on the presence of a toxic substance. The diagram illustrates the change in color as distinguished by using a pH indicative dye. As can be seen from Example 3, since it is possible to regulate the pH change by controlling the concentration of an external electron donor added, the method of the present invention can be also effectively applied to such a system as described in FIG 7, which shows the color of a pH indicative dye changed depending on the pH change.

Referring to FIG 7, it is possible to estimate the presence of toxicity and intensity thereof by comparing the color change of a pH indicative dye with that of a control. In comparison with the initial color of a pH indicative dye, the less the color change is, the stronger the toxicity of a test sample is, while the more the color change is similar to that of a control, the less the toxicity of a test sample is.

As described above, the present invention has the excellent advantages as follows.

First of all, since the method for detecting the effect of a toxic substance using SMP measures the rate of pH change occurring outside the SMP by employing the phenomenon that an electron transport system of SMP is inhibited by the presence of a toxic substance, it can apply to various measuring systems as well as detect the presence of toxicity and intensity thereof within a short time.

Further, according to the method for detecting the effect of a toxic substance using SMP, it is capable of detecting the toxicity regardless of the kind of a toxic substance, thereby making it possible to apply in many fields.

## Claims

1. A method for detecting the effect of a toxic substance using sub-mitochondrial particle (SMP), which comprises
contacting a sub-mitochondrial particle with a sample;
adding an external electron donor to the sample;
measuring a pH change; and
correlating the pH change with a concentration of the toxic substance in the sample.

2. The method for detecting the effect of a toxic substance using SMP according to claim 1, wherein the external electron donor is at least one selected from the group consisting of NADH and FADH₂ .

3. The method for detecting the effect of a toxic substance using SMP according to claim 1, wherein the pH change is measured as an electrical signal.

4. The method for detecting the effect of a toxic substance using SMP according to claim 3, wherein the electrical measurement is carried out by measuring one or more signals selected from the group consisting of electric current, electric potential, impedance and capacitance.

5. The method for detecting the effect of a toxic substance using SMP according to claim 1, wherein the pH change is measured as a color change by using a pH indicator.

## Patentansprüche

1. Verfahren zum Erfassen der Wirkung einer toxischen Substanz unter Verwendung submitochondrialer Partikel (SMP), das umfasst:
Herstellen von Kontakt eines submitochondrialen Partikels mit einer Probe;
Hinzufügen eines externen Elektronenspenders zu der Probe;
Messen einer pH-Änderung; und
Korrelieren der pH-Änderung zu einer Konzentration der toxischen Substanz in der Probe.

2. Verfahren zum Erfassen der Wirkung einer toxischen Substanz unter Verwendung submitochondrialer Partikel nach Anspruch 1, wobei der externe Elektronenspender wenigstens ein aus der Gruppe ausgewählter ist, die aus NADH und FADH₂ besteht.

3. Verfahren zum Erfassen der Wirkung einer toxischen Substanz unter Verwendung submitochondrialer Partikel nach Anspruch 1, wobei die pH-Änderung als ein elektrisches Signal gemessen wird.

4. Verfahren zum Erfassen der Wirkung einer toxischen Substanz unter Verwendung submitochondrialer Partikel nach Anspruch 3, wobei die elektrische Messung ausgeführt wird, indem ein oder mehrere Signal/e gemessen wird/werden, das/die aus der Gruppe ausgewählt wird/werden, die aus elektrischem Strom, elektrischem Potenzial, lmpedanz und Kapazität besteht.

5. Verfahren zum Erfassen der Wirkung einer toxischen Substanz unter Verwendung submitochondrialer Partikel nach Anspruch 1, wobei die pH-Änderung als eine Farbänderung unter Verwendung eines pH-Indikators gemessen wird.

## Revendications

1. Procédé pour détecter l'effet d'une substance toxique en utilisant une particule sous-mitochondriale (SMP), qui comprend
la mise en contact d'une particule sous-mitochondriale avec un échantillon ;
l'addition d'un donneur d'électrons externe à l'échantillon;
la mesure d'un changement de pH ; et
la corrélation du changement de pH avec une concentration de la substance toxique dans l'échantillon.

2. Procédé pour détecter l'effet d'une substance toxique en utilisant une SMP selon la revendication 1, dans lequel le donneur d'électrons externe est au moins un donneur choisi dans le groupe constitué par NADH et FADH₂.

3. Procédé pour détecter l'effet d'une substance toxique en utilisant une SMP selon la revendication 1, dans lequel le changement de pH est mesuré sous la forme d'un signal électrique.

4. Procédé pour détecter l'effet d'une substance toxique en utilisant une SMP selon la revendication 3, dans lequel la mesure électrique est effectuée en mesurant un ou plusieurs signaux choisis dans le groupe constitué par un courant électrique, un potentiel électrique, une impédance et une capacité.

5. Procédé pour détecter l'effet d'une substance toxique en utilisant une SMP selon la revendication 1, dans lequel le changement de pH est mesuré sous la forme d'un changement de couleur en utilisant un indicateur de pH.
